Europäisches Patentamt

European Patent Office

Office européen des brevets

(19)

(11) Veröffentlichungsnummer: **0 389 977**
**A1**

## (12) EUROPÄISCHE PATENTANMELDUNG

(21) Anmeldenummer: **90105520.2**

(22) Anmeldetag: **23.03.90**

(51) Int. Cl.⁵: **C12N 1/00, C12N 1/38**

(30) Priorität: **29.03.89 YU 641/89**

(43) Veröffentlichungstag der Anmeldung:
**03.10.90 Patentblatt 90/40**

(84) Benannte Vertragsstaaten:
**AT BE CH DE DK ES FR GB GR IT LI LU NL SE**

(71) Anmelder: **Gajic, Branko R. Dr.**
**Bulevar Lenjina 105/7**
**YU-11070 Novi Beograd(YU)**

(72) Erfinder: **Gajic, Branko R. Dr.**
**Bulevar Lenjina 105/7**
**YU-11070 Novi Beograd(YU)**

(74) Vertreter: **Gudel, Diether, Dr. et al**
**Patentanwälte Dr. V. Schmied-Kowarzik**
**Dipl.-Ing. G. Dannenberg Dr. P. Weinhold Dr.**
**D. Gudel Dipl.-Ing. S. Schubert Dr. P. Barz**
**Grosse Eschenheimer Strasse 39**
**D-6000 Frankfurt am Main 1(DE)**

(54) **Nährstoff auf der Basis pflanzlicher Rohstoffe der Familie Caryophyllaceae für Mikroorganismen, insbesondere Pilze und Bakterien.**

(57) Beschrieben wird ein Nährstoff auf der Basis pflanzlicher Rohstoffe der Familie Caryophyllaceae für Mikroorganismen, insbesondere Pilze und Bakterien (Hefe).

EP 0 389 977 A1

**Nährstoff auf der Basis pflanzlicher Rohstoffe der Familie Caryophyllaceae für Mikroorganismen, insbesondere Pilze und Bakterien**

Die Erfindung betrifft einen Nährstoff auf der Basis pflanzlicher Rohstoffe der Familie Caryophyllaceae für Mikroorganismen, insbesondere für Pilze, Bakterien, Hefe, Penicillin, Schimmelpilze, das Myzelium von Speisepilzen und die Fruktifikation von Speisepilzen. Mit dem erfindungsgemäßen Nährstoff behandelte Mikroorganismen können vorteilhaft beispielsweise in der Nahrungsmittelindustrie (Bäckerhefe und dergl.) und auch in der industriellen Mikrobiologie eingesetzt werden. Ein weiteres Einsatzgebiet ist die pharmazeutische Industrie.

Die europäische Offenlegungsschrift 283 744 des Anmelders beschreibt einen Bioregulator, der aus bestimmten Bestandteilen pflanzlicher Rohstoffe der Familie Caryophyllaceae zusammengesetzt ist. Dieser bekannte Bioregulator ist ein Pflanzenhilfsmittel mit breitem Wirkungsspektrum. Er wird entweder dem Saatgut beigemischt oder dient auch zur Blattbehandlung. In jedem Fall werden nur Nutzpflanzen mit ihm behandelt. Die Behandlung von Mais, Zuckerrüben und Sonnenblumen ist dort ausdrücklich erwähnt.

Die noch nicht veröffentlichte deutsche Patentanmeldung P 39 04 169.7 des Anmelders schildert die Verwendung dieses Bioregulators zum Verringern der krebserregenden, tabakspezifischen Nitrosamine im Tabak. Auch hier werden also Kulturpflanzen, nämlich Tabak, mit diesem Bioregulator behandelt.

Demgegenüber liegt der Erfindung die Aufgabe zugrunde, den Einsatzzweck eines Nährstoffs auf der Basis pflanzlicher Rohstoffe der Familie Caryophyllaceae zu erweitern.

Die Erfindung besteht in der Verwendung eines Nährstoffs auf der Basis pflanzlicher Rohstoffe der Familie Carryophillaceae für Mikroorganismen, insbesondere Pilze und Bakterien (Hefe).

Der Erfindung liegt die überraschende Erkenntnis zugrunde, daß die vorstehend definierten Nährstoffe auch für Mikroorganismen geeignet sind, d.h. für Lebewesen einer ganz anderen Kategorie als der Einsatzzweck der Bioregulatoren nach dem Stand der Technik. Es stand nicht zu erwarten, daß diese Nährstoffe sich auch erfolgreich bei Mikroorganismen einsetzen lassen, weil bekanntlich die pflanzenphysiologischen Vorgänge in Mikroorganismen vollständig verschieden sind von denen in Kulturpflanzen.

Versuche haben ergeben, daß insbesondere zwei Arten dieser Nährstoffe erfolgreich eingesetzt werden können, wobei die in Patentanspruch 2 angegebenen Zusammensetzung für die erste Art, insbesondere für die unmittelbare Herstellung von Mikroorganismen (Hefe, Schimmelpilze, Myzilium von Speisepilzen sowie Fruktifikation von Speisepilzen), eingesetzt werden kann.

Die zweite Gruppe der Nährstoffe, deren Zusammensetzung in Patentanspruch 3 definiert ist, läßt sich insbesondere vorteilhaft in der Nahrungsmittelindustrie und in der industriellen Mikrobiologie anwenden.

Patentanspruch 4 beschreibt eine bevorzugte Verfahrensführung zur Herstellung der Zusammensetzung nach Patentanspruch 2. Dasselbe gilt für das Verfahren nach Patentanspruch 5 bezüglich der Zusammensetzung nach Anspruch 3.

Die Erfindung wird im folgenden näher erläutert.

Wie erwähnt, liegt der Erfindung das Problem zugrunde, das Wachstum und die Entwicklung von Mikroorganismen anzuregen und deren Stoffwechsel zu stimulieren, nach Möglichkeit verbunden mit einer Verbesserung qualitativer Eigenschaften sowie Steigerung quantitativer Verwendungsmöglichkeiten der Mikroorganismen.

Grundlegende Ergebnisse der durchgeführten Untersuchungen sowie die gewonnenen physiologisch aktiven Präparate können überwiegend zur Lösung vieler moderner Probleme angewendet werden, und stellen deshalb eine wichtige Möglichkeit zur Regulierung der Qualität und Produktivität nützlicher Mikroorganismen in industrieller Mikrobiologie sowie in Technologien der Fertigprodukte in der Lebensmittelindustrie dar, in welchen die nützlichen Mikroorganismen als notwendige Komponente verwendet werden.

Weitere Vorteile dieser Erfindung liegen darin, daß die Primärrolle der physiologisch aktiven, nach dem in dieser Anmeldung enthaltenen Verfahren gewonnenen Präparate, in der Verbesserung qualitativer Eigenschaften und Steigerung quantitativer Verwendungsmöglichkeiten von niederen Nutzpflanzen (Hefen, Schimmel, Pilze) in industrieller Mikrobiologie und in der Lebensmittelindustrie zu sehen ist. Sekundäre Rolle hat die anregende Einwirkung physiologisch aktiver Präparate auf den Stoffwechsel der genannten niederen Pflanzen mit dem Zweck, die qualitativen und quantitativen Eigenschaften der aus dem Organismus ausgeschiedenen Metaboliten zu erhöhen.

Auf diese Weise, durch Erhöhung quantitativer und Verbesserung qualitativer Eigenschaften der niederen Nutzpflanzen, bzw. Hefen, Schimmel, Pilze und auch bei deren wichtigen Metaboliten - unter der Einwirkung von physiologisch aktiven Präparaten - stellen diese in Technologien der Lebensmittelindustrie Komponenten und Fertigprodukte von besserer Qualität und auch qualitativ bessere Fertigprodukte in Technologien der industriellen Mikrobiologie dar. In dem Sinne werden durch diese Erfindung positive Verwendungsmöglichkeiten der gewonnenen physiologisch aktiven Präparate pflanzlicher Herkunft in einer Reihe von Technologien geboten, insbesondere jedoch

A) In Technologien zur unmittelbaren Produktion von niederen Nutzpflanzen und/oder deren Metaboliten, und zwar:

1. der Hefe, 2. der Schimmel und 3. des Myzeliums der Nutzpilze und bei der Fruktifikation der Speisepilze.

B) In Technologien der Endprodukte in der Nahrungsmittel industrie und industrieller Mikrobiologie:

1. im Bäckereigewerbe, 2. in der Industrie der alkoholischen und alkoholfreien Getränke und 3. in der pharmazeutischen Industrie.

Im mit A) bezeichneten Bereich spiegelt sich die grundlegende positive Einwirkung der aufgrund des in dieser Anmeldung beschriebenen Verfahrens gewonnenen physiologisch aktiven Präparate durch die Herstellung verschiedener Arten nützlicher Kulturhefen der Gattung Saccharomycetes wieder, und zwar sind es:

a) Bäckerhefe, b) Bierhefe, c) alkoholische Hefe und d) Nähr-oder Fütterhefe.

Der wesentliche stimulative Einfluß von physiologisch aktiven Präparaten pflanzlicher Herkunft kommt durch Einwirkung auf physiologische Wachstums- und Entwicklungsparameter der nützlichen Gattungen von Kulturhefen zum Ausdruck, wobei die Anzahl der überlebenden Zellen bei der Einpflanzung auf verschiedenen Typen von festen und flüssigen Nährboden sowie durch spätere schnellere und stärkere Zellenvermehrung in aeroben und anaeroben Züchungsverhältnissen erhöht wird.

In aeroben Verhältnissen wirkt sich der positive Einfluß von physiologisch aktiven Präparaten auf eine Zuwachssteigerung der Hefe-Biomase und auf größere spezifische Wachstumsgeschwindigkeit sowie auf die Verkürzung des Vermehrungs-Lebenszyklus aus. Im Laufe der Durchführung dieses Verfahrens ist festgestellt worden, daß die konkrete Zuwachserhöhung der Biomasse von Bäckerhefe, die in der Melasse als Nährboden gezüchtet wurde, im Durchschnitt 7,3 % betrug, wobei sich die Erhöhung der spezifischen Wachstumsgeschwindigkeit sogar auf 11 % belief. Da die spezifische Wachstumsgeschwindigkeit Maßstab für Melassenqualität ist, kann man sagen, daß durch Zugabe von physiologisch aktiven Präparaten die Melassenqualität veredelt und verbessert wird, was für Melassenhersteller sowie für Endverbraucher von zweifelloser Bedeutung ist.

In aeroben Züchtungsverhältnissen kommen positive Einwirkungen physiologisch aktiver Präparate durch stärkere fermentative Aktivität der Hefe, bzw. auch durch Stoffwechseldynamik der Fermentation von Kohlendioxid ($CO_2$) zum Ausdruck. In Abhängigkeit vom Nährboden und Hefegattung bewegen sich diese Erhöhungen der alkoholischen fermentativen Aktivität im Bereich von 24,2 % bis 46,8 %.

Um die Bedeutung des Verfahrens, das Gegenstand der Erfindung ist, besser zu verstehen, muß betont werden, daß die Anwendung physiologisch aktiver Präparate pflanzlicher Herkunft eine Neuerung bei der Veresserung der Hefequalität und -quantität sowie eine neue technologische Maßnahme in der Produktion darstellt. Die bisherige Anwendung künstlich hergestellter Wuchsstoffe wie BIOTIN, INOSITOL, Pantothensäure u. ähnl., hat neben ihrer Vorteile auch eine Reihe von Nachteilen gezeigt, vor allem vom wirtschaftlichen Standpunkt gesehen. Das heißt, die Hersteller haben einen sehr hohen Preis zu zahlen. Durch Anwendung der relativ billigen physiologisch aktiven Präparate pflanzlicher natürlicher Herkunft, kann man - je nach verwendeten Hefegattungen und Nährböden - die Wuchsstoffe - unter Steigerung der Herstellungs- und Wirtschaftlichkeitseffekte - um 50 % reduzieren oder sogar völlig ersetzen.

Es muß ebenfalls betont werden, daß die Einwirkung von physiologisch aktiven Präparaten pflanzlicher Herkunft auf nützliche Kultur-Schimmelpilze, bzw. Verbesserung von qualitativen und quantitativen Eigenschaften der Basisgattungen, von großer Bedeutung ist. Dabei werden auch positive Ergebnisse der Einwirkung auf die Gruppe Fungi imperfecti und auf ihren wichtigsten Vertreter, Penicillium chrysogenum, hervorgehoben. Durch Einwirkung der physiologisch aktiven Präparate pflanzlicher Herkunft wird auch eine größere Biomasse der Basisgattung, und bei Laborfermentation ein hohes Niveau der Penicillintiter G oder V, nebst einer kürzeren Ruhephase (Produktion der Biomasse) gewonnen. Die Anwendung von physiologisch aktiven Präparaten pflanzlicher Herkunft ist auch in der pharmazeutischen Industrie eine neue technologische Maßnahme.

Die Anwendung physiologisch aktiver Präparate hat auch bei der Myzeliumherstellung von Speisepilzen der Gattung Agaricus und Pleurotus, und insbesondere Pleurotus ostreatus und Pleurotus pulmonarius, positive Ergebnisse zur Folge gehabt. Es wurden dabei vergrößerte Wachtumszonen des Myzeliums um 38,4 - 59,6 %, je nach Arten gewonnen. Die genannten Stimulationen wurden auf defizienten Nährböden gewonnen, was auf die Möglichkeit hinweist, das teuere Natursubstrat unter Zugabe der physiologisch aktiven Präparate durch ein billigeres zu ersetzen und annähernd gleiche Leistung bei Myzeliumherstellung zu erreichen.

Physiologisch aktive Präparate pflanzlicher Herkunft, die nach dem in dieser Anmeldung enthaltenen Verfahren gewonnen werden, können zur Erhöhung der Fruktifikation von Speisepilzen eingesetzt werden, die je nach Gattung und Art 9 - 16 % betragen kann. Darüber hinaus wird auch Protein- und Trockensubstanz erhöht.

In dem unter B) aufgeführten Bereich spielen die physiologisch aktiven Präparate pflanzlicher Herkunft ebenfalls eine positive Rolle, in erster Linie im Bäckereigewerbe, und innerhalb dieses vor allem in der Technologie der Brotherstellung. Dabei haben sie eine Doppelrolle bzw. -funktion:

a) Additivfunktion zur Verbesserung der Mehl- bzw. Teigqualität und

b) Funktion der physiologisch aktiven Agenzien zur Verbesserung der Aktivität von Bäckerhefe.

a) In ihrer Additivfunktion zur Verbesserung der Mehl- bzw. Teigqualität wirken die physiologisch aktiven Präparate im allgemeinen auf Qualitätsverbesserung von minderwertigen Mehlsorten, deren technologische Qualität für die Herstellung von Brot-Qualitätssorten nicht entsprechend ist, positiv ein. In dem Sinne wird der Einfluß auf die Erhöhung der amylolitischen Aktivität bzw. Abnahme der maximalen Viskosität bei amylographischen Untersuchungen hervorgehoben. Es wird möglich gemacht, durch stärkere wärmebedingte Zerstörung der Kristallit-Stärkestruktur unter Verbindung von Substanzen aus den Präparaten, was sich auf Mehlsorten, die an amylolitischen Enzymen (Alfa- und Beta-Amilase) arm siond, günstig auswirkt.

Ein weiterer wichtiger Moment ist die Inhibition der proteolytischen Aktivität in Mehlsorten mit hohem Gehalt der proteolytischen Enzyme durch Zugabe des aufgrund dieser Erfindung erhaltenen Präparates. Dieser positive Effekt ist durch Bildung neuer -S-S-Verbindungen zwischen makromolekularen polypeptiden Reihen und Hemmung der Aktivatoren proteolytischer Enzyme (Glutation) durch die im Präparat enthaltenen Verbindung möglich.

b) Die Funktion physiologisch aktiver Präparate im Sinne einer Verbesserung der Bäckerhefe-Aktivität kann durch Beurteilung des Brotes nach den bestehenden Vorschriften verfolgt werden, wobei ein positiver Einfluß auf Geruch und Geschmack der Kruste und des Brotleibes sowie auf die Verbesserung der chemischen Zusammensetzung (Verteilung und Menge von essentiellen und nicht essentiellen Aminosäuren u.ä.) des Brotes, und auf sonstige Parameter seiner Qualtätit (Löchrigkeit, Porosität, Höhe und Umfang des Brotes) festgestellt wurde.

Die beiden genannten Wirkungsrichtungen der physiologisch aktiven Präparate hatten eine gemeinsame positive Folge -nämlich qualitativ besseres Brot vom Standpunkt der organoleptischen Einschätzung, chemischen Zusammensetzung und Verlängerung der Frischedauer des Brotes (48 Stunden länger).

Günstige Einwirkung der physiologisch aktiven Präparate auch auf sonstige Hefesorten, wie Bierhefe, alkoholische Hefe u.a. ist bereits betont worden. Die genannten Präparate können jedoch auch in der Technologie der Biererzeugung sowie bei der Herstellung verschiedener Weinsorten verwendet werden. Diese Präparate können auch zur Verbesserung der Endproduktqualität eingesetzt werden.

Die nach dem in dieser Anmeldung enthaltenen Verfahren gewonnenen physiologisch aktiven Präparate wirken auch günstig auf verschiedene Gattungen von Penicillium chrysogenum in der Herstellungstechnologie von Penicillin G oder V und haben eine Erhöhung der Biomasse Penicillium chrysogenum in Ruhephase zur Folge, wodurch sie verkürzt wird, so daß die Exponentialphase der Bildung von Penicillin G oder V früher beginnt, in Abhängigkeit von der Technologie und dem gewünschten Endprodukt. Dabei wird Penicillintitar für Basisgattungen zwischen 20 - 25 g/l um 6 - 11 % im Durchschnitt, umgerechnet auf Trockensubstanz, erhöht, was vom Herstellungs- und Wirtschaftsstandpunkt ohne Zweifel sehr bedeutend ist.

Die physiologisch aktiven Präparate pflanzlicher Herkunft sind für Menschengesundheit und Umwelt völlig unschädlich. Nämlich die Ergebnisse des Gehalts von organochlorischen Insektiziden und Schwermetallen, deren aktute Toxizität, akute orale $LD_{50}$, die mehr als

$$\frac{5.000 \ \mathbf{mg \ des \ Präparates}}{\mathbf{kg \ T.M.}}$$

beträgt, und akuten dermalen $LD_{50}$, die größer ist als

$$\frac{4.000 \ \mathbf{mg \ des \ Präparates,}}{\mathbf{kg \ T.M.}}$$

festgestellt wurde, sowie die Ergebnisse der Haut- und Schleimhaut der Rattenaugen, die darauf hinwiesen, daß die physiologisch aktiven Präparate, die für genannte Zwecke bestimmt sind, keine Irritation der Augenhaut oder leichte Irritation der -schleimhaut bei Ratten hervorrufen und Ergebnisse der histologischen Untersuchungen von Versuchstieren weisen darauf hin, daß die physiologisch aktiven Präparate pflanzlicher Herkunft, die für die Anwendung in industrieller Mikrobiologie und Nahrungsmittelindustrie bestimmt sind, für Menschengesundheit untoxisch und unschädlich sind und daß sie für genannte Anwendungszwecke geeignet sind.

In Abhängigkeit davon, wo die physiologisch aktiven Präparate pflanzlicher Herkunft eingesetzt werden, in industrieller Mikrobiologie oder Nahrungsmittelindustrie, sind durch diese Erfindung zwei grundlegende technologische, untereinander unabhängige Verfahren entwickelt, so daß man auch zwei physiologisch aktive Basispräparate, nämlich das "Gro-Caryophyllin II" (siehe die Patentansprüche 2 und 4) und "Gro-Caryophyllin III" (siehe die Patentansprüche 3 und 5) bekommen kann, wobei das "Gro-Caryophyllin II" eine basische aktive Substanz für abgeleitete physiologisch aktive Endpräparate ist, die in Technologien zur unmittelbaren Herstellung von niederen Nutzpflanzen (Hefe, Schimmelpilze und Myzelium von Speisepilzen sowie Fruktifikation von Speisepilzen) eingesetzt werden kann. Das "Gro-Caryophyllin III" stellt in diesem Sinne eine basische Aktivsubstanz für abgeleitete Endprodukte physiologisch aktiver Präparate, die in der Technologie der Nahrungsmittelindustrie und industriellen Mikrobiologie (wie oben unter B) aufgeführt) Anwendung finden.

Für die Durchführung des in dieser Anmeldung genannten Verfahrens werden als pflanzliche Grundrohstoffe Pflanzen aus der Familie Caryophyllaceae eingesetzt, und zwar: Herniaria L., Spergula L., Ortega L., Arenaria L., Sagina L., Holosteum L., Lychnis githago Scop. und sonstige Pflanzengattungen aus der Familie Caryophyllaceae.

Man kann verschiedene Teile der genannten Pflanzen verwenden, d.h. Blüten, Samen, Stiele, Blätter, und zwar solche, die aus unterschiedlichen Vegetationsphasen stammen. Die ausgewählten Pflanzenteile werden in speziellen Mühlen, die besondere Konstruktion haben und für besondere Zwecke bestimmt sind, vereinzelt gemahlen, d.h. jede benutzte Art bzw. Komponente wird gesondert gemahlen. Beim Mahlen ist darauf zu achten, daß die Granulation der Teilchen folgende Durchmesser hat:
a) $d = 5.10^{-5} - 1,5.10^{-4}$ m und b) $d = 1.10^{-6} - 5.10^{-5}$ m. Das Verfahren wird in Chargen ausgeführt. Es gibt zwei Grundverfahren, und zwar eines zur Gewinnung von "Gro-Caryophyllin II" und das andere zur Gewinnung von "Gro-Caryophyllin III".

Nach dem ersten Verfahren (siehe Anspruch 4) werden die Rohstoffe nacheinander und unter ununterbrochenen Mischerumdrehungen den Bioreaktor eingeworfen, und zwar: 25 % Herniaria L., 14 % Spergula L., 35 % Ortega L., 7 % Arenaria L., 8 % Sagina L., 6 % Holosteum L., 5 % sonstige Pflanzenkomponenten aus Caryophyllaceae-Familie. Die Teilchen mit dem Durchmesser $d = 5.10^{-5} - 1,5.10^{-4}$ m sind in einer Menge von 9,58 %, und die mit dem Durchmesser $d = 1.10^{-6} \div 5.10^{-5}$ m sind in einer Menge von 83,92 % vorhanden. Die Umdrehungszahl des Mischers beträgt 100/min. Das Verfahren wird bei einer Temperatur von 10° C durchgeführt. Die Mischzeit beträgt 30 min. Nach Mischbeendigung und Extraktion wird die Masse zum Trocknen geführt, das bei einer Temperatur von 30° C stattfindet. Nach dem Trocknen werden die Granula bis zum Pulver homogenisiert. Während des Mischvorgangs, der im Bioreaktor durchgeführt wird, erfolgt die Zugabe eines Extraktionsmittels, das Polareigenschaften hat (Aethanol, Wasser). Deshalb beträgt das Wasser im fertigen Präparat 7,13 %.

Nach dem zweiten Verfahren (siehe Patentanspruch 5) werden Rohstoffe in folgenden Mengen in den Bioreaktor eingeworfen: 20 % Herniaria L., 10 % Spergula L., 30 % Ortega L., 5 % Arenaria L., 6 % Sagina L., 4 % Holosteum L., 10 % Lychnis githago Scop. und 15 % sonstige Pflanzenkomponenteaus der Familie Caryophyllaceae. Die Teilchen, deren Durchmessser $d = 5.10^{-5} - 1,5.10^{-4}$ m beträgt, machen 13,25 % und Teilchen mit Durchmesser $d = 1.10^{-6} - 5.10^{-5}$ m, 82,15 % der Mischung.

Im zweiten Verfahren ist die Umdrehungszahl des Mischers wesentlich größer und beträgt 600/min. Der Mischvorgang erfolgt bei einer wesentlich höheren Temperatur, nämlich bei 50° C. Im Unterschied zur ersten Ausführungsart beträgt die Mischzeit 60 min. Während des Mischvorgangs wird der Mischung im Bioreaktor ein Extraktionsmittel mit Polareigenschaften, wie z.B. Wasser oder Aethanol, zugefügt.

Nach Mischbeendigung, während welcher auch die Extraktion der Nasse erfolgt, wird bei beiden Verfahren die Masse auf eine Temperatur von 50° C getrocknet. Nach der Trocknung erfolgt die Homogenisierung von Granula bis zum Pulver. Das Fertigprodukt enthält 5,32 % Feuchtigkeit.

Das nach dem ersten oder zweiten Verfahren gewonnene physiologisch aktive Basispräparat wird in der Folge in Aufbewahrungsräumen bei einer Temperatur von 20° C zum Absitzenlassen eingelagert. Das Absitzen dauert bis zu 12 Monaten.

Aufgrund der organoleptischen Eigenschaften ist das nach der ersten Ausführungsart gewonnene Präparat eine pulverisierte, grau-weiße und angenehm riechende Substanz. Gegenüber diesem ist das aufgrund der zweiten Ausführungsart gewonnene Präparat grau-gelblicher Farbe und riecht auch angenehm, jedoch etwas stärker. Im ersten Fall beträgt der Feuchtigkeitsgehalt 7,13 % und im zweiten Fall 5,32 %. Von den genannten Eigenschaften hängt auch das spezifische Volumen als wichtige Charakteristik zur Auswahl und Projektierung der Verpackung sowie zur Dosierung des pulverigen Präparates ab und beträgt bei dem nach der ersten Ausführungsart gewonnenen Präparat 1,54l/kg und bei dem nach der zweiten Ausfüh-

rungsart gewonnenen Präparat 1,58 l/kg.

Weitere Unterschiede zwischen dem ersten und dem zweiten Präparat bestehen aus folgendem: Die Löslichkeit des nach der ersten Ausführungsart gewonnenen Präparates im Wasser beträgt bei einer Temperatur von 20°C 47,48 %. Der Anteil der ungelösten Substanz beträgt 49,17 %. Der Gehalt von leichtflüchtigen Komponenten bei einer Temperatur von 105°C beträgt 3,35 %. Je nach Konzentration der Lösung bewegt sich der pH-Wert zwischen 6,26 und 6,82. Bei dem nach zweiter Ausführungsart gewonnenen Präparat beträgt die Löslichkeit im Wassere bei 20°C 25,36 %. Der ungelöste Anteil beträgt 25,36 %, der Gehalt von leichtflüchtigen Stoffen ist 7,09 %, während sich der pH-Wert im Bereich von 6,38 bis 6,94 bewegt.

Die gewonnenen Präparate können je nach Bedarf oder Anwendungs zwecken mit organischen oder anorganischen Trägern im Verhältnis 1:50 und sogar bis 1:1000 gemischt werden; sie können aber auch ohne die Träger verwendet werden. Alle Arten der gravimetrischen und volumentrischen Dosierung und Verpackung Dosierung und Verpackung sind praktisch möglich.

Das pulverförmige Präparat kann fünf Jahre verwendet werden, während bei der Lösung diese Frist drei Jahre beträgt. Die nach den in dieser Anmeldung enthaltenen Verfahren gewonnenen physiologisch aktiven Präparate können einmal oder mehrmals verwendet werden, was von der Züchtungstechnologie der niederen Pflanzen (Hefen, Schimmel, Pilze) oder von Technologien der Nahrungsmittelindustrie bzw. industriellen Mikrobiologie abhängt. Eine mögliche und gewünschte Anwendungsart des Präparates besteht aus der Zugabe des Pulvers oder der Lösung der festen oder flüssigen Nährböden während deren Bereitung (alle Typen der festen Nährböden auf Agar-Agar-, Melasse-, Malz-, usw. Basis), wobei Endprodukte der Nahrungsmittelindustrie (Bäckergewerbe, Industrie der alkoholischen und alkoholfreien Getränke) und pharmazeutischen Industrie (Penicillin G, Penicillin V, usw.) gewonnen werden.

**Ansprüche**

1. Verwendung eines Nährstoffs auf der Basis pflanzlicher Rohstoffe der Familie Caryophyllaceae für Mikroorganismen, insbesondere Pilze und Bakterien (Hefe).

2. Nährstoff nach Anspruch 1,
**dadurch gekennzeichnet,**
daß er die folgenden Hauptbestandteile, ggf. mit üblichen Zusätzen, enthält:
a.) etwa 25 Gew. % Herniaria,
b.) etwa 14 Gew. % Spergula,
c.) etwa 35 Gew. % Ortega,
d.) etwa 7 Gew. % Arenaria,
e.) etwa 8 Gew. % Sagina,
f.) etwa 6 Gew. % Holosteum und
g.) etwa 0 bis 5 Gew. % sonstige pflanzliche Bestandteile aus der Familie Caryophyllaceae,
wobei die angegebenen Gernichtsprozente um jeweils ± 10 % nach oben oder unten schwanken können.

3. Nährstoff nach Anspruch 1,
**dadurch gekennzeichnet,**
daß er die folgenden Hauptbestandteile, ggf. mit üblichen Zusätzen, enthält:
a.) etwa 20 Gew. % Herniaria,
b.) etwa 10 Gew. % Spergula,
c.) etwa 30 Gew. % Ortega,
d.) etwa 5 Gew. % Arenaria,
e.) etwa 6 Gew. % Sagina,
f.) etwa 4 Gew. % Holosteum,
g.) etwa 0 bis 15 Gew. % sonstige pflanzliche Bestandteile aus der Familie Caryophyllaceae und
h.) etwa 10 Gew. % Lychnig githago Scop.,
wobei die angegebenen Gewichtsprozente um jeweils ± 10 % nach oben oder unten schwanken können.

4. Verfahren zur Herstellung eines Nahrstoffs nach Anspruch 2,
**dadurch gekennzeichnet,**
daß die pflanzlichen Rohstoffe pulverisiert, in einem Mischer gemischt und mit Hilfe eines Extraktionsmittels extrahiert werden, wobei die Umdrehungszahl des Mischers etwa 100 pro Minute beträgt, das Verfahren bei etwa 10°C durchgeführt wird und die Mischzeit etwa 30 Minuten beträgt, worauf die so entstandene Masse getrocknet und pulverisiert wird.

5. Verfahren zur Herstellung eines Nährstoffs nach Anspruch 3,
**dadurch gekennzeichnet,**
daß die pflanzlichen Rohstoffe pulverisiert, in einem Mischer gemischt und mit Hilfe eines Extraktionsmittels extrahiert werden, wobei die Umdrehungszahl des Mischers etwa 600 pro Minute beträgt, das Verfahren bei etwa 50°C durchgeführt wird und die Mischzeit etwa 60 Minuten beträgt, worauf die so entstandene Masse getrocknet und pulverisiert wird.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 037 454  (PAUL HABERL)<br>* Ansprüche *<br>--- | 1 | C 12 N   1/00<br>C 12 N   1/38 |
| D,A | EP-A-0 283 744<br>(PATENTVERWERTUNGSGESELLSCHAFT<br>BÜRGELICHEN RECHTS)<br>* Ganzes Dokument *<br>----- | 1-5 | |

**RECHERCHIERTE SACHGEBIETE (Int. Cl.5)**

A 01 N
A 21 D
C 12 N

Der vorliegende Recherchenbericht wurde für alle Patentansprüche erstellt

| Recherchenort | Abschlußdatum der Recherche | Prüfer |
|---|---|---|
| DEN HAAG | 02-07-1990 | HUBER A. |